(19) Europäisches Patentamt
European Patent Office
Office européen des brevets

(11) Veröffentlichungsnummer: **0 089 592**
**B1**

(12) # EUROPÄISCHE PATENTSCHRIFT

(45) Veröffentlichungstag der Patentschrift: **26.02.86**

(21) Anmeldenummer: **83102478.1**

(22) Anmeldetag: **14.03.83**

(51) Int. Cl.⁴: **C 07 C 45/68**, C 07 C 49/233, C 07 C 49/203, C 07 C 49/207, C 07 C 49/213, C 07 C 49/84, C 07 C 49/04

(54) **Verfahren zur Herstellung von Methylketonen.**

(30) Priorität: **24.03.82 DE 3210725**

(43) Veröffentlichungstag der Anmeldung: **28.09.83 Patentblatt 83/39**

(45) Bekanntmachung des Hinweises auf die Patenterteilung: **26.02.86 Patentblatt 86/9**

(84) Benannte Vertragsstaaten: **AT BE CH DE FR GB IT LI NL**

(56) Entgegenhaltungen:
**FR - A - 1 029 064**
**FR - A - 1 384 137**
**US - A - 3 983 175**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Lantzsch, Reinhard, Dr., Heymannstrasse 32, D-5090 Leverkusen 1 (DE)**
Erfinder: **Elbe, Hans-Ludwig, Dr., Dasnöckel 59, D-5600 Wuppertal 11 (DE)**

**EP 0 089 592 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein neues vorteilhaftes Verfahren zur Herstellung von teilweise bekannren Methylketonen, die als Zwischenprodukte für die Synthese von Pflanzenschutzmitteln verwendet werden können.

Es ist bereits bekannt geworden, daß Ketone und Aldehyde unter Phasen-Transfer-Katalyse-Bedingungen alkyliert werden können. Aldehyde dürfen dabei jedoch nur ein α-ständiges Wassersroffarom enthalten, da sonst aufgrund von Dialkylierung und O-Alkylierung die Ausbeuten auf unter 20 % sinken (vergleiche Chemistry and Industry 1978, 732). Ketone geben nur dann gute Ausbeuten an gewünschrem Monoalkylierungsprodukt, wenn eine zusätzliche Akrivierung durch einen Arylrest, wie z.B. im Phenylaceton ($C_6H_5$-$CH_2$-CO-$CH_3$), vorhanden ist In anderen Fällen, wie beispielsweise bei Acerophenon oder Aceton erfolgt die Bildung von Gemischen aus Mono-, Di- und O-alkylierten Produkten, wobei die Ausbeuten an Mono-alkyliertem Produkt gering sind ( vergleiche Tetrahedron Letters 18, 1351-52 (1971) und Journal of Organic Chemistry of the USSR 17329 (1981)).

Weiterhin werden in der Patentliteratur Verfahren zur Herstellung von substituierten Keto-Derivaten beschrieben: Dabei werden z.B. organische Halogenide mit einer Keto-Verbindung umgesetzt, die ein austauschbares Wasserstoffatom besitzt; die Synthese wird in Gegenwart von Aminen durchgeführt (vgl. US-PS 3 983 175). Ferner kann man in ähnlicher Weise in alpha-Stellung zur Keto-Gruppe Alkylierungen in Gegenwart von basischen Katalysatoren durchführen (vgl. FR-PS 1 384 137). Beiden Verfahren haften Nachteile an; so wird beim erstgenannten Verfahren in Gegenwart von reichlich Wasser gearbeitet, worunter die Ausbeute leidet, während beim zweitgenannten Verfahren Üerrschüssiges Keton entfernt werden muß.

Es wurde gefunden, daß man die teilweise bekannten Methylketone der allgemeinen Formel

$$R^1 - CH_2 - \underset{R^3}{\overset{R^2}{\underset{|}{\overset{|}{C}}}} - CO - CH_3 \qquad (I)$$

in welcher

$R^1$ für Alkyl mit 1 bis 4 und für Alkenyl und Alkinyl mit jeweils 2 bis 4 Kohlenstoffatomen steht, und ferner für gegebenenfalls durch Fluor, Chlor, Brom, Methyl, Ethyl, Isopropyl, tert.-Butyl, Methoxy, Methylthio, Trifluormethyl, Trifluormethoxy, Trifluormethylthio, Dimethylamino und Ethylmethylamino substituiertes Phenyl steht, und schließlich für gegebenenfalls durch Fluor, Chlor, Brom, Methyl und/oder Etyl substituiertes Pyridinyl, Pyrimidinyl, Furyl oder Thiophenyl steht, substituiertes Pyridinyl, Pyrimidinyl, Furyl oder Thiophenyl steht,

$R^2$ und $R^3$ für Methyl stehen oder beide gemeinsam mit de für Cyclopropyl stehen, vorteilhaft in guten Ausbeuten erhält, wenn man Methylsek.-alkyl-ketone der Formel

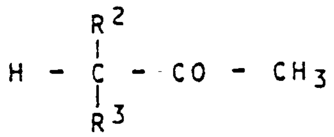 (II)

in welcher

$R^2$ und $R^3$ die oben angegebene Bedeutung haben,

mit Halogeniden der Formel

$$R^1 - CH_2 - X \qquad (III)$$

in welcher

$R^1$ die oben angegebene Bedeutung hat und
X für Halogen steht,

im Verhältnis von 1 bis 2 Mole Keton der Formel (II) auf 1 Mol Halogenid der Formel (III) in Gegenwart von pulverisiertem Kalium- oder Natriumhydroxid und in Gegenwart eines Verdünnungsmittels, sowie in Gegenwart eines Phasentransferkatalysators umsetzt.

Es ist im Hinblick auf den Stand der Technik als überraschend zu bezeichnen , daß die erfindungsgemäße Umsetzung in der gewünschten Weise verläuft und keine Di-, Tri-, Tetraoder O-Alkylierungen auftreten bzw. keine Folgereaktionen und Kondensationen der Methyl-sek.-Alkylketone der Formel (II) mit sich selbst zu beobachten sind.

Das erfindungsgemäße Verfahren liefert die Methylketone der Formel (I) vorteilhaft in sehr guten Ausbeuten.

Die nach dem erfindungsgemäßen Verfahren erhältlichen Methyl-ketone sind durch die Formel (I) definiert.

Verwendet man beispielsweise Isopropyl-methyl-keton und 4-Chlorbenzylchlorid als Ausgangsstoffe, so kann der Reaktionsablauf des erfindungsgemäßen Verfahrens durch das folgende Formelschema wiedergegeben werden:

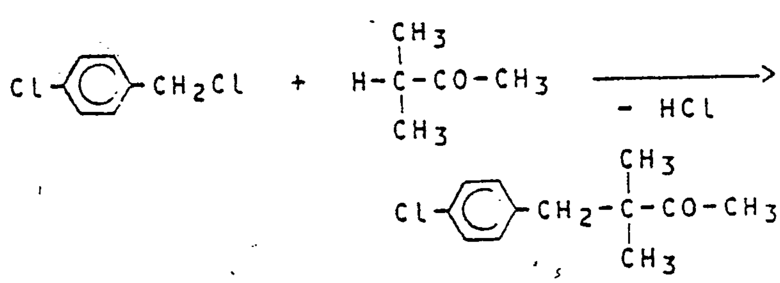


Die für das erfindungsgemäße Verfahren als Ausgangsstoffe zu verwendenden Methyl-sek.-alkyl-ketone sind durch die Formel (II) allgemein definiert.

Die Methyl-sek.-Alkyl-ketone der Formel (II) sind allgemein bekannte Verbindungen der organsichen Chemic.

Die außerdem für das erfindungsgemäße Verfahren als Ausgangsstoffe zu verwendenden Halogenide sind durch die Formel (III) allgemein definiert. In dieser Formel steht X steht vorzugsweise für Chlor.

Die Halogenide der Formel (III) sind ebenfalls allgemein bekannte Verbindungen der organischen Chemie.

Als Verdünnungsmittel kommen für die erfindungsgemäße Umsetzung vorzugsweise gegen Basen stabile, inerte organische Lösungsmittel in Frage. Hierzu gehören vorzugsweise (cyclo)aliphatische und gegebenenfalls chlorierte aromatische Kohlenwasserstoffe, wie Benzol, Xylol, Chlorbenzol, Dichlorbenzol, Cyclohexan, Petrolether, Benzin, Pentan, Hexan und insbesondere Toluol.

Als Basen verwendet man für die erfindungsgemäße Umsetzung pulverisiertes Kalium- und Natriumhydroxid. Bevorzugt wird technisches Kaliumhydroxid eingesetzt.

Als Phasentransferkatalysatoren können alle üblicherweise verwendbaren Derivate von Ammoniumsalzen eingesetzt werden, wie beispielsweise Triethylbenzylammonium-chlorid, Tetrabutylammonium-iodid und insbesondere Tetrabutylammonium-bromid oder -chlorid sowie Trioctylmethylammoniumchlorid.

Die Reaktionstemperaturen können bei der erfindungsgemäßen Umsetzung in einem größeren Bereich variiert werden. Im allgemeinen arbeitet man zwischen 0 und 150°C, vorzugsweise zwischen +20 und 130° C.

Bei der Durchführung der erfindungsgemäßen Umsetzung setzt man auf 1 bis 2 Mol Keton der Formel (II) 1 Mol Halogenid der Formel (III) und katalytische Mengen an Katalysator ein. In einer bevorzugten Form der Durchführung werden Katalysator und Base im Lösungsmittel vorgelegt und das Halogenid der Formel (III) im Genisch mit dem Keton der Formel (II) Langsam bei der Reaktionstemperatur zugetropft. Die erfindungsgemäß herstellbaren Methylketone der Formel (I) sind teilxeise bekannt (vergleiche z.B. Bull. Soc. Chin. France 1970, 912 oder C.R.Séances Acad. Sci., Ser. C 269 (1969) 18 1052-1055); sie können als Zwischenprodukte zur Herstellunhgvon fungiziden, insektiziden herbiziden und,/ oder pflanzenwachstumsregulierenden Verbindungen eingesetzt werden.

Beispielsweise erhält man aus den Methylketonen der Formel (Ia) nach dem folgenden Reaktionsschema fungizid wirksame Verbindungen der Formel (IV) (vgl. die Deutsche Patentanmeldung P 30 48 266 vom 20.12.1980 [ LeA 20 763]):

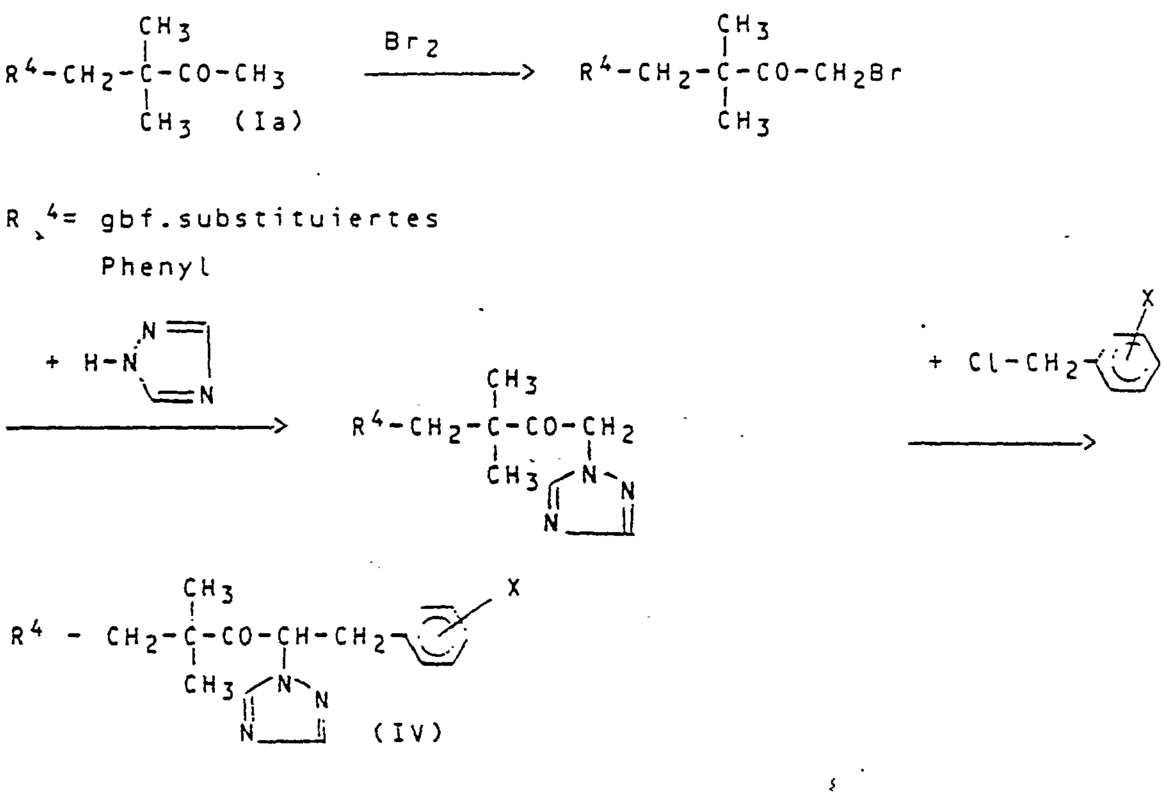


(Ia)

$R^4$= gbf.substituiertes Phenyl

(IV)

X = z.B. Wasserstoff,Halogen

## Herstellungsbeispiele

**Beispiel 1**

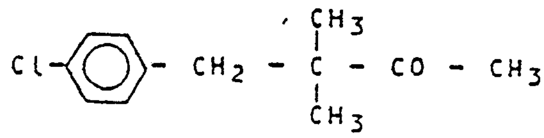

In 1 l Toluol suspendiert man 254,4g (4 Mol)pulverisiertes technisches Kaliumhydroxid (88 %-ig), fügt 40g Tetrabutylammoniumbromid zu und tropft dann ein Gemisch von 644 g (4 Mol) 4-Chlorbenzylchlorid und 430 g (5 Mol) Methyl-isopropyl-keton langsam bei 85°C zu. Zur Vervollständigung der Reaktion wird nach beendetem Zutropfen noch 3 Stunden bei 85°C gerührt. Nach dem Abkühlen filtriert man das Kaliumchlorid ab und wäscht das Filtrat neutral. Durch fraktionierte Destillation werden 732,5g (87 % der Theorie) 1-(4-Chlorphenyl)-2,2-dimethyl-3-butanon vom Siedepunkt 87-9O°C/ 0,05 mbar erhalten.

**Beispiel 2**

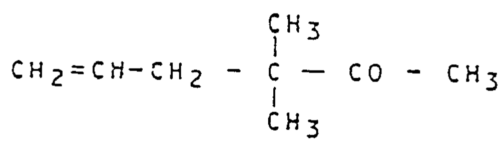

Zu einer Mischung aus 250ml Toluol, 86g (1 Mol) Methylisopropyl-keton, 69g (0,9 Mol) Allylchlorid, sowie 10g Terrabutylammoniumbromid werden 63,6g (1 Mol) pulverisiertes Kaliumhydroxid (88 %-ig) zudosiert. Die Reaktion ist exotherm. Nach beendeter Zugabe wird weitere 12 Stunden bei 100°C gerührt. Man arbeitet entsprechend Beispiel 1 auf und erhält 77g (67,9 % der Theorie) 4,4-Dimethyl-1-hexen-5-on vom Siedepunkt 45-46°C/12 mbar. oder

Man suspendiert 63,6g (1 Mol) pulverisiertes technisches Kaliumhydroxid (88%-ig) in 200 ml Toluol und fügt 10g Trioctylmethylammoniumchlorid zu. Dann tropft man eine Mischung aus 76,5g (1 Mol) Allylchlorid und 129g (1,5 Mol) Methyl-isopropyl-keton so zu, daß die Temperatur 70°C nicht übersteigt. Man rührt noch 4 Stunden bei 70°C nach und arbeitet wie in Beispiel 1 auf. Man erhält 96g (76 % der Theorie) 4,4-Dimethyl-1-hexen-5-on vom oben genannten Siedepunkt.

**Beispiel 3**

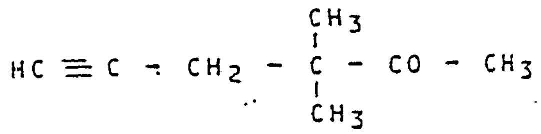

In 250 ml Toluol werden 70g (1,1 Mol) pulverisiertes tecknisches Kaliumhydroxid (88%-ig) und 10g Tetrabutylammoniumbromid vorgelegt. Dann wird eine Mischung aus 119g (1 Mol) Propargylbromid und 103,2g (1,2 Mol) Methylisopropyl-keton so zugetropft, daß die Temperatur nicht über 45° C steigt. Man rührt noch 12 Stunden bei 45° C nach und arbeitet entsprechend Beispiel 1 auf. Man erhält 67g (54% der Theorie) 4,4-Dimethyl-1-hexin-5-on vom Siedepunkt 55° C/ 12 mbar.

**Beispiel 4**

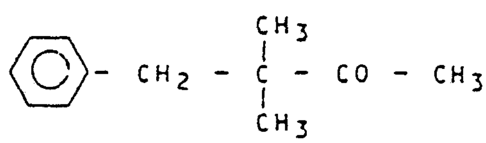

516g (6 Mol) Methyl-isopropyl-keton, 759g ( 6 Mol) Benzylchlorid und 60g (0,186 Mol) Tetrabutylammoniumbromid werden in 1 l Toluol gelöst und auf 100° C erwärmt. Bei dieser Temperatur werden 420g (7,49 Mol) gepulvertes Kaliumhydroxid langsam zudosiert. Man läßt das Reaktionsgemisch 12 Stunden bei 100° C rühren, kühlt ab und versetzt mit 1,5 l Wasser. Die organische Phase wird abgetrennt, über Natriumsulfat getrocknet und fraktioniert destilliert. Man erhält 507g 3,3-Dimethyl-4-phenyl-2-butanon vom Siedepunkt 82° C/0,05 mbar.

In entsprechender Weise können die Verbindungen der allgemeinen Formel(I)

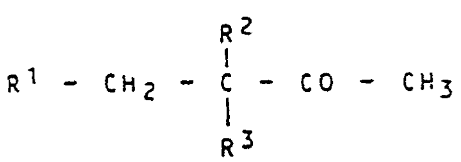

(I)

erhalten werden:

| Bsp. Nr. | $R^1$ | $R^2$ | $R^3$ | Physikal. Konstante |
|---|---|---|---|---|
| 5 | 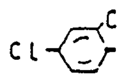 | $CH_3$ | $CH_3$ | Kp:90-95°C/ 0,06 mbar |
| 6 | 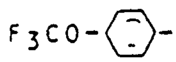 | $CH_3$ | $CH_3$ | Kp:56°C/ 0,05 mbar |
| 7 | 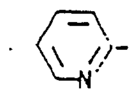 | $CH_3$ | $CH_3$ | Kp:75-80°C/ 0,05 mbar |
| 8 | 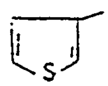 | $CH_3$ | $CH_3$ | Kp:66-70°C/ 0,15 mbar |
| 9 | $C_2H_5$ | $CH_3$ | $CH_3$ | Kp:140-46°C |
| 10 | $n-C_3H_7$ | $CH_3$ | $CH_3$ | Kp:59-65°C/ 18 mbar |
| 11 | $CH_3-CH=CH-$ | $CH_3$ | $CH_3$ | Kp:66-71°C/ 28 mbar |
| 12 | 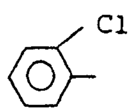 | $CH_3$ | $CH_3$ | Kp: 70-75°C/ 0,05 mbar |
| 13 | 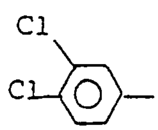 | $CH_3$ | $CH_3$ | Kp: 110-15°C/ 0,1 mbar |
| 14 | 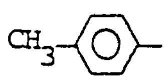 | $CH_3$ | $CH_3$ | Kp: 72-77°C/ 0,05 mbar |
| 15 | 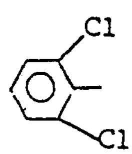 | $CH_3$ | $CH_3$ | Kp: 100-0,5°C/ 0,1 mbar |
| 16 | 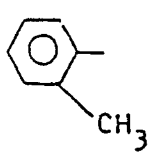 | $CH_3$ | $CH_3$ | Kp: 87°C 0,4 mbar |
| 17 | 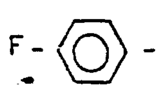 | $CH_3$ | $CH_3$ | Bp: 67-70°C 0,05 mbar |

**Herstellung eines Folgeproduktes**

a)

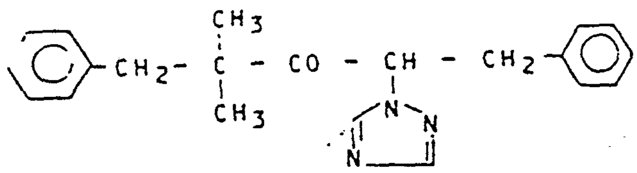

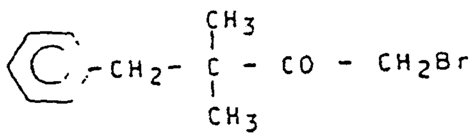

100g (0,57 Mol) 3,3-Dimethyl-4-phenyl-2-butanon (Beispiel 5) werden in 0,8 l Chloroform gelöst und bei Raumtemperatur langsam mit 29 ml (91,9 ; 1,14 Mol) Brom versetzt. Man läßt eine Stunde bei Raumtemperatur nachrühren und engt ein. Man erhält 145,5g (quantitativ) 1-Brom-3,3-dimethyl-4-phenyl-2-butanon, das ohne Isolierung direkt weiter umgesetzt wird.

b)

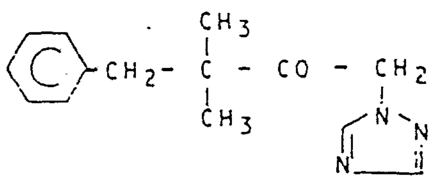

74g (0,29 Mol) 1-Brom-3,3-dimethyl-4-phenyl-2-butanon,40 g (0,58 Mol) 1,2,4-Triazol und 80g Kaliumcarbonat werden in 700 ml Aceton gelöst und 3 Stunden unter Rückfluß erhitzt. Danach läßt man abkühlen, saugt vom anorganischen Rückstand ab und engt das Filtrat ein. Der Rückstand wird aus Erher umkristallisiert. Man erhält 34,1g (48,4 % der Theorie) 3,3-Dimethyl-4-phenyl-1-(1,2,4-triazol-1-yl)-2-butanon vom Schmelzpunkt 79°C.

c)

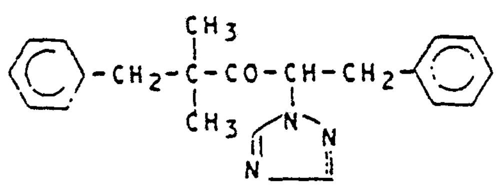

48,6g (0,2 Mol) 3,3-Dimethyl-4-phenyl-1-(1,2,4-triazol-1-yl)-2-butanon, 27,9g (0,22 Mol) Benzylchlorid und 12,3 g (0,22 Mol) Kaliumhydroxid in 15 ml Wasser werden in 300 ml Dimethylsulfoxid gelöst. Man läßt 8 Stunden bei 50°C nachrühren und gießt das Reaktionsgemisch anschlienend auf Wasser. Man extrahiert mit Essigester. Die organische Phase wird eingeengt und der Rückstand säulenchromatographisch (Kieselgel/Essigester: Cyclohexan = 3:1) gereinigt.Man erhält 54,5g (81,8 % der Theorie) 1,5-Bisphenyl-2,2-dimethyl-4-(1,2,4-triazol-1-yl)-3-pentanon vom Schmelzpunkt 45-50°C.

**Patentansprüche**

1. Verfahren zur Herstellung von Methylketonen der allgemeinen Formel

$$R^1-CH_2-\underset{R^3}{\overset{R^2}{C}}-CO-CH_3 \qquad (I)$$

in welcher

$R^1$ für Alkyl mit 1 bis 4 und für Alkenyl und Alkinyl mit jeweils 2 bis 4 Kohlenstoffatomen steht, und ferner für gegebenenfalls durch Fluor, Chlor, Brom, Methyl, Ethyl, Isopropyl, tert.-Butyl, Methoxy, Methylthio, Trifluormethyl, Trifluormethoxy, Trifluormethylthio, Dimethylamino und Ethylmethylamino substituiertes Phenyl steht, und schließlich für gegebenenfalls durch Fluor, Chlor, Brom, Methyl und/oder Ethyl substituiertes Pyridinyl, Pyrimidinyl, Furyl oder Thiophenyl steht,

$R^2$ und $R^3$ für Methyl stehen oder beide gemeinsam mit dem Kohlenstoffatom, an da sie gebunden sind, für Cyclopropyl stehen,

dadurch gekennzeichnet, daß man Methyl-sek.-alkylketone der Formel

$$H-\underset{R^3}{\overset{R^2}{C}}-CO-CH_3 \qquad (II)$$

in welcher $R^2$ und $R^3$ die oben angegebene Bedeutung haben, mit Halogeniden der Formel

$$R^1-CH_2-X \qquad (III)$$

in welcher

$R^1$ die oben angegebene Bedeutung hat und

X für Halogen steht,

im Verhältnis von 1 bis 2 Mole Keton der Formel (II) auf 1 Mol Halogenid der Formel (III) in Gegenwart von pulverisiertem Kalium- oder Natriumhydroxid und in Gegenwart eines Verdünnungsmittels, sowie in Gegenwart eines Phasentransferkatalysators umsetzt.

2. Verfahren gemäß Anspruch 1, dadurch gekennzeichnet, daß man die Umsetzung im Temperaturbereich zwischen 0 und 150°C durchführt.

3. Verfahren gemäß Ansprüchen 1 und 2, dadurch gekennzeichnet, daß man die Umsetzung im Temperaturbereich zwischen +20 und 130°C durchführt.

4. Verfahren gemäß Anspruch 1, dadurch gekennzeichnet, daß man als Phasentransferkatalysator Derivate von Ammoniumsalzen einsetzt.

**Claims**

1. Process for the preparation of methyl ketones of the general formula

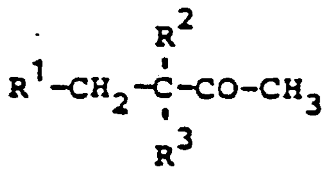 (I)

in which

$R^1$ represents alkyl with 1 to 4 and alkenyl and alkinyl each with 2 to 4 carbon atoms, and also represents optionally fluorine-, chlorine-, bromine-, methyl-, ethyl-, isopropyl-, tert.-butyl-, methoxy-, methylthio-, trifluoromethyl-, trifluoromethoxy-, trifluoromethylthio-, dimethylamino- and ethylmethylamino-substituted phenyl, and finally represents optionally fluorine-,chlorine-, bromine-, methyl- and/or ethyl-substituted pyridinyl, pyrimidinyl, furyl or thiophenyl, and $R^2$ and $R^3$ represent methyl or both, together with the carbon atom to which they are bonded, represent cyclopropyl, characterised in that methyl sec.-alkyl ketones of the formula

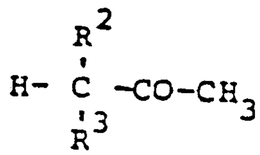 (II)

in which

$R^2$ and $R^3$ have the abovementioned meaning, are reacted with halides of the formula

$$R^1-CH_2-X \quad (III)$$

in which

$R^1$ has the abovementioned meaning and

X represents halogen, in a ratio of 1 to 2 mols of the ketone of the formula(II) to 1 mol of the halide of the formula (III) in the presence of powdered potassium or sodium hydroxide and in the presence of a diluent, and in öthe presence of a phase-transfer catalyst.

2. Process according to Claim 1, characterised in that the reaction is carried out in the temperature range between 0 and 150°C.

3. Process according to Claims 1 and 2, characterised in that the reaction is carried out in the temperature range between +20 and 130°C.

4. Process according to Claim 1, characterised in that derivatives of ammonium salts are used as the phase-transfer catalyst.

**Revendications**

1. Procédé de fabrication de méthylcétones de formule générale :

$$R^1-CH_2-C(R^2)(R^3)-CO-CH_3 \qquad (I)$$

dans laquelle

$R^1$ représente un alcoyle ayant 1 à 4 et un alcényle et alcinyle ayant chacun 2 à 4 atomes de carbone, en outre un phényle éventuellement substitué par du fluor, chlore, brome, méthyle, éthyle, isopropyle, t-butyle, méthoxy, méthylthio, trifluorométhyle, trifluorométhoxy, trifluorométhylthio, diméthylamino et ethylméthylamino, et finalement un pyridinyle, pyrimidinyle, furyle ou thiophényle éventuellement substitué par du fluor, brome, méthyle et/ou éthyle,

$R^2$ et $R^3$ représentent un méthyle ou, à eux deux conjointement avec l'atome de carbone auquel ils sont attachés, un cyclopropyle, caractérisé en ce qu'on fait reagir des méthyl-secalcoyl-cétones de formule

$$H-C(R^2)(R^3)-CO-CH_3 \qquad (II)$$

dans laquelle

$R^2$ et $R^3$ ont la signification indiquée plus haut, avec des halogènures de formule

$$R^1-CH_2-X \qquad (III)$$

dans laquelle

$R^1$ a la signification indiquée plus haut et X représente de l'halogène,

dans la proportion de 1 à 2 moles de cétone de formule (II) pour 1 mole de l'halogènure de formule (III) en présence d'hydroxyde de potassium ou de sodium pulvérisé et en présence d'un diluant, de même qu'en présence d'un catalyseur de transfert de phase.

2. Procedé selon la revendication 1, caractérisé en ce qu'on exécute la réaction dans l'intervalle de température compris entre 0 et 150° C.

3. Procédé selon les revendications 1 et 2, caractérisé en ce qu'on exécute la réaction dans l'intervalle de température compris entre +20 et 130° C.

4. procédé selon la revendication 1, caractérisé en ce qu'on utilise comme catalyseur de transfert de phase des dérivés de sels d'ammonium.